Europäisches Patentamt

European Patent Office

Office européen des brevets

(11) Numéro de publication: **0 112 784 B1**

## FASCICULE DE BREVET EUROPEEN

(45) Date de publication de fascicule du brevet:
06.03.91

(51) Int. Cl.⁵: **A61K 39/015**, G01N 33/569

(21) Numéro de dépôt: 83402547.0

(22) Date de dépôt: **27.12.83**

(54) **Fractions polypeptidiques inductrices d'anticorps protecteurs contre des parasites du paludisme et compositions immunogènes les contenant.**

(30) Priorité: 27.12.82 FR 8221817
27.12.82 FR 8221818
28.07.83 FR 8312496
21.12.83 FR 8320510

(43) Date de publication de la demande:
04.07.84 Bulletin 84/27

(45) Mention de la délivrance du brevet:
06.03.91 Bulletin 91/10

(84) Etats contractants désignés:
**AT BE CH DE FR GB IT LI LU NL SE**

(56) Documents cités:
EP-A- 0 062 924
GB-A- 2 099 300

CHEMICAL ABSTRACTS, vol. 94, no. 25, 22 juin 1981, ref. no. 206968w, p. 447, Columbus, Ohio (US)

NATURE, vol. 289, no. 5795, 22 janvier 1981, MacMillan Journals Ltd., Chesham, Bucks (GB); L.H. PERRIN et al.:"Inhibition of P. falciparum growth in human erythrocytes by monoclonal antibodies", pp. 301-303

NATURE, vol. 289, no. 5793, 1/8 janvier 1981, MacMillan Journals Ltd., Paris (FR); W.A. SIDDIQUI et al.:"Use of a synthetic adjuvant in an effective vaccination of monkeys against malaria", pp. 64-66

CHEMICAL ABSTRACTS, vol. 98, no. 19, 9 mai 1983, ref. no. 158880v, pp. 361-362, Columbus, Ohio (US)

(73) Titulaire: **INSTITUT PASTEUR**
**25-28, rue du Docteur Roux**
**F-75724 Paris Cédex 15(FR)**

(72) Inventeur: **Les inventeurs ont renoncé àleur désignation**

(74) Mandataire: **Gutmann, Ernest**
**S.C. Ernest Gutmann - Yves Plasseraud 67,**
**boulevard Haussmann**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

L'invention concerne des fractions polypeptidiques inductrices d'anticorps protecteurs contre des parasites du paludisme, ainsi que des compositions immunogènes les contenant, susceptibles de conduire à la production de vaccins pour l'homme et l'animal. Elle est également relative aux anticorps spécifiques induits in vivo par ces fractions.

L'expression "fractions polypeptidiques" est utilisée dans le texte qui suit pour la commodité du langage. Elle ne doit pas être interprétée de façon restrictive. Il peut s'agir en fait de fractions de protéines ou de fractions immunogéniques ayant une structure chimique distincte, par exemple contenant des antigènes saccharidiques ou glycoprotéiniques ou glycopeptidiques.

En d'autres termes, l'expression "polypeptide" désigne en fait tout constituant peptidique ou protéinique, notamment du type protéine ou glycoprotéine, tel que ceux qui peuvent être obtenus à partir de Plasmodium falciparum ou de parasites du paludisme susceptibles d'infecter l'homme ou des primates.

On sait que chez l'homme ou l'animal qui a été atteint par le paludisme et qui est devenu résistant, il existe des anticorps contre des formes érythrocytaires du parasite. L'existence de ces anticorps peut être démontrée par la protection qui peut temporairement être conférée à un animal non immunisé, sensible ou "naïf", par transfert passif à celui-ci d'un immunsérum d'un animal immunisé ou d'immunoglobulines purifiées à partir de ce sérum.

Plusieurs tentatives d'isolement d'un principe immunogène protecteur à partir d'extraits de diverses espèces de parasites responsables d'une forme correspondante du paludisme ont été décrites dans la littérature technique. Plus récemment, la technologie des hybridomes a permis d'isoler de nouveaux anticorps susceptibles d'être mis en oeuvre dans l'analyse des antigènes des constituants des parasites du paludisme.

C'est ainsi que YOSHIDA et Coll. (1980) Science, 207, 71, ont isolé un antigène capable d'induire une réponse immunitaire protectrice chez des rongeurs. D'autres laboratoires ont également mis en oeuvre la technologie des hybridomes dans le but d'isoler des anticorps dirigés contre certaines espèces de Plasmodium falciparum, capables notamment d'infecter la souris. Des lignées d'hybridomes secréteurs d'anticorps, capables d'inhiber la croissance du parasite en culture ont été décrites par PERRIN et Coll. (1981), Nature, 289-301. Ces auteurs ont montré que les anticorps monoclonaux produits avaient des propriétés d'inhibition en culture d'une souche de P . falciparum capable d'infecter la souris.

Un principe antigénique présenté comme pouvant être utilisé pour la constitution de vaccins contre le paludisme a été décrit dans la demande de brevet européen n° 0071705. Ce principe actif, obtenu notamment à partir de parasites de l'espèce Plasmodium , présente les caractéristiques suivantes :

1°) sa masse moléculaire est de $1,8 \times 10^5$ à $2,5 \times 10^5$ ;

2°) il est associé avec les membranes des formes schizontes érythrocytaires ou mérozoïtes du parasite et

3°) ce principe antigénique est susceptible d'être fragmenté,au sein même des érythrocytes,infectés en fragments plus petits possédant les mêmes propriétés antigéniques;

ledit antigène ou les fragments formés à partir de celuici étant associés aux surfaces membranaires des mérozoïtes.

Parmi les Plasmodium cités, certains étaient des Plasmodium falciparum d'origine humaine.

La demande de brevet européen décrit également un procédé pour isoler ce principe antigénique, procédé qui comprend la solubilisation d'érythrocytes comprenant des formes schizontes du parasite Plasmodium, la mise en contact de la matière solubilisée avec des anticorps monoclonaux spécifiques de la protéine antigénique recherchée, de préférence préalablement fixés sur un support solide, pour former un complexe anticorps-antigène, l'élimination des protéines ou polypeptides non engagés dans le complexe ou non fixés et la récuparation de la protéine antigénique à partir du complexe anticorps-antigène. Les anticorps monoclonaux avaient été obtenus à partir d'hybridomes sélectionnés parmi des hybrides cellulaires, eux-mêmes obtenus par fusion entre des cellules de souris préalablement immunisées avec le parasite choisi et des cellules de myélomé.

Des résultats démontrant le caractère protecteur du principe antigénique ainsi obtenu à l'égard de la souris ont effectivement été rapportés.

Bien que prometteurs, ces résultats doivent être considérés avec précaution. En effet, les parasites (paludiques ) infectieux pour l'homme et le primate ne le sont pas en général pour la souris et vice versa. On ne peut donc exclure que dans les systèmes antérieurement décrits, le système immunitaire de la souris ait reconnu des déterminants antigéniques contenus dans les extraits utilisés pour l'immunisation, n'étant cependant pas aptes à induire des anticorps réellement protecteurs pour le primate ou l'homme. Certes des antigènes obtenus étaient susceptibles d'être reconnus par des immunsérums humains in vitro, par exemple dans des expériences d'immuno-précipitation. Des résultats quant à leur activité protectrice chez le singe ou l'homme n'ont pas été rapportés.

L'invention a pour but de fournir un principe actif perfectionné immunogène susceptible d'être obtenu à partir des diverses espèces de parasites connues ou susceptibles d'être reconnues pour être capables d'infecter l'homme ou le primate, qui présente une aptitude plus certaine à la vaccination humaine contre la malaria ou le paludisme. Elle a encore pour but l'obtention d'un procédé permettant d'obtenir de tels principes immunogènes.

La composition immunogène selon l'invention contient un ou plusieurs polypeptides contenus dans des parasites du paludisme, **ces polypeptides** étant plus particulièrement aptes à réagir avec des anticorps protecteurs provenant de singes résistant aux parasites humains du paludisme et notamment à des espèces de Plasmodium falciparum.

L'invention met à profit le fait qu'il est en effet possible d'adapter au singe Saimtri Sciureus (ou Aotus trivirgatus, ou encore Rhésus) des parasites infectieux pour l'homme, notamment Plasmodium falciparum et d'obtenir une infection comparable, en tous points identique à l'infection humaine. Les animaux peuvent être rendus résistants par une infection expérimentale, suivie d'un traitement approprié, notamment par la quinine. Cette résistance est directement liée à l'apparition chez ces animaux d'anticorps protecteurs. On rappelle que le parasite injecté chez un premier singe, puis ayant fait l'objet de passages successifs dans plusieurs singes, s'y adapte au point de devenir virulent pour le singe, et plus particulièrement chez le singe splénectomisé.

En particulier, l'invention concerne des fractions polypeptidiques obtenues à partir de P. falciparum comprenant les polypeptides ayant des poids moléculaires moyennes s'étageant d'environ 72.000 à environ 140.000,
- qui sont inductrices notamment chez le singe, et plus particulièrement le singe Saimiri Sciureus, d'anticorps actifs contre des parasites du paludisme, plus particulièrement de Plasmodium falciparum ou des parasites qui présentent les mêmes caractéristiques biologiques essentielles,
- qui sont reconnues par des sérums ou autres compositions d'immunoglobulines provenant d'animaux, notamment de singes Saimiri Sciureus, résistants au parasite, ces sérums ou autres compositions d'immunoglobulines étant capables, par transfert passif in vivo à des animaux sensibles au parasite, de les protéger contre ledit parasite

On remarquera que ces fractions polypeptidiques sont également reconnues par les anticorps provenant d'individus adultes résidant en zone endémique et présentant un degré de résistance élevé à P. falciparum. Le pouvoir portecteur du sérum de tels individus a déjà été montré lors d'expériences de transfert passif chez des enfants atteints de paludisme (COHEN et Coll., 1961, Nature, 192:733).

Avantageusement, les compositions immunogènes de l'invention sont obtenues à partir de la souche de Plasmodium falciparum qui a fait l'objet d'un dépôt à la Collection Nationale des Cultures de Micro-organismes de L'INSTITUT PASTEUR de Paris (C.N.C.M.) sous le n° FUPC I-212 le 23 décembre 1982.

L'invention a plus particulièrement pour objet toute composition immunogène contenant un ou plusieurs polypeptides choisis parmi des polypeptides ayant des poids moléculaires de l'ordre de 50.000 à 140.000, ces polypeptides étant caractérisés:
- en ce qu'ils sont reconnus par des anticorps protecteurs contre les parasites du paludisme, ces anticorps étant contenus dans des sérums, ascites ou autres compositions d'immunoglobulines, provenant de singes Saimiri Sciureus rendus résistants à la souche de P.falciparum déposée à la C.N.C.M. sous le numéro I-212, cette résistance étant obtenue par infection de ces singes par ladite souche de P.falciparum (devenue virulente par passages successifs de ladite souche chez plusieurs singes Saimiri Sciureus splenectomisés) suivie d'un traitement par la quinine, ces sérums, ascites ou autres compositions d'immunoglobulines, étant capables par transfert passif in vivo à des singes receveurs splénectomisés sensibles, de les protéger contre l'infection par ladite souche de P.falciparum, lorsque ceux-ci ont reçu au préalable l'injection de $50.10^6$ cellules, parasitées par ladite souche de P.falciparum, ces cellules étant elles-mêmes obtenues à partir de singes Saimiri Sciureus splénectomisés et infectés par ladite souche de P.falciparum, et au moment où l'infection était aiguë et en phase ascendante,
- en ce qu'ils sont inducteurs, chez le singe Saimiri Sciureus splenectomisés, de la production d'anticorps protecteurs contre les parasites du paludisme dans les conditions suivantes:
. les singes sus-mentionnés sont inoculés trois fois à trois semaines d'intervalle avec les polypeptides par injections sous-cutanées,
. trois semaines après la troisième injection, les animaux sont infectés avec $50.10^6$ globules rouges parasités par la souche de P.falciparum susmentionnée, par voie intraveineuse.

Un premier groupe de fractions polypeptidiques préférées possédant les propriétés sus-indiquées sont en outre caractérisées par des poids moléculaires moyens de 72.000, 75.000-76.000, 80.000, 83.000, 85.000 ou 90.000 daltons. Des fractions polypeptiques particulièrement préférées de l'invention présentent des poids moléculaires de l'ordre de 75.000 $\pm$ 5.000.

Un autre groupe de fractions polypeptidiques préférées de l'invention concerne des compositions polypeptidiques caractérisées par des polypeptides présentant les poids moléculaires suivants: 90.000, 95.000, 100.000, 105.000, 110.000, 115.000, 120.000 et 130.000 daltons, au sein d'un ensemble complexe s'étageant de 83.000 à 140.000 daltons. En particulier, l'invention concerne des fractions polypeptidiques ayant une masse moléculaire moyenne de l'ordre de 100.000 $\pm$ 10.000.

L'invention concerne également plus particulièrement les fractions davantage purifiées présentant des poids moléculaires respectivement contenus dans les intervalles 72.000 $\pm$ 2.000 et 90.000 $\pm$ 5.000 daltons. L'ensemble des polypeptides de poids moléculaires qui précèdent peuvent être mis en évidence par incorporation métabolique d'acides aminés marqués, par exemple la méthionine$^{35}$S.

Une troisième catégorie de fractions polypeptidiques ayant des caractéristiques immunogènes protectrices sont caractérisées par des poids moléculaires moyens de l'ordre de 50.000 $\pm$ 5.000. L'invention concerne plus spécialement parmi ces dernières fractions celles qui ne peuvent pas être mises en évidence par l'incorporation métabolique d'acides aminés marqués, tels que la méthionine$^{35}$S.

Les masses moléculaires indiquées dans ce qui précède résultent des mesures comparatives dans un système électrophorétique, d'une part, des distances de migration des fractions concernées et, d'autre part, des distances de migration mesurées, dans des conditions semblables de peptides de poids moléculaires connus, plus particulièrement des IgG humaines, la sérumalbumine bovine (BSA), l'ovalbumine de poulet, la phosphorylase B et la myosine. Avantageusement les polypeptides et peptides utilisés sont marqués radioactivement ou non radioactivement, par exemple à l'isothyocyanate de fluorescéine.

L'invention concerne encore des préparations davantage purifiées, caractérisées par leur capacité à réagir avec l'anticorps monoclonal du type IgG$_2$a, secrété par l'hybridome déposé à la C.N.C.M. le 20 décembre 1983 sous le n° I-271 et obtenu par fusion cellulaire de myélome (souche Sp2/O-Ag 14) et de cellules spléniques de souris Balb/c immunisées avec des fractions de poids moléculaires moyens de 100.000 daltons obtenus à partir de la susdite souche FUPC I-212. Cet anticorps monoclonal réagit spécifiquement avec un antigène de poids moléculaire 90.000, spécifiquement reconnu par des immunoglobulines protectrices obtenues à partir d'un singe immunisé Saimiri Sciureus.

Plasmodium falciparum est bien connu des spécialistes. Les schizontes et les mérozoïtes de ce parasite ont déjà été utilisés pour réaliser des vaccins expérimentaux contre la malaria, notamment chez le singe [MITCHELL et al, (1977), Lancet, i, 1335, et SIDDIQUI, W.A. (1977), Science, 197, 388 (1977)]. Les résultats biologiques observés chez le singe et rapportés dans ce dernier article intitulé "An effective immunization of experimental monkeys against the human malaria parasite, Plasmodium falciparum" peuvent être considérés comme significatifs et extrapolables aux résultats qui pourraient être observés chez l'homme.

La capacité de ce même parasite d'infecter les singes Saimiri Sciureus est bien connue aussi (GYSIN et al, 1980, J. Parasitol. 66 : 1003). Il a également été montré que cet animal manifeste une réponse humorale élevée (GYSIN et al, 1982, Ann. Immunol. (Institut Pasteur) 133 D: 95) et que des anticorps protecteurs sont produits dans la phase chronique de l'infection (GYSIN et al, 1982, Parasite Immunol. 4 : 421).

L'invention concerne donc toutes préparations obtenues à partir de parasites infectieux pour l'homme ou le primate, susceptibles de réagir avec ces anticorps protecteurs (sérum, ascites provoquées chez l'animal ou immunoglobulines davantage purifiées à partir de ce sérum), obtenues à partir de singes Saimiri Sciureus immunisés.

La présence des anticorps protecteurs dans le sérum de l'animal, particulièrement le singe, peut être appréciée par la capacité induite par le sérum de l'animal immunisé (ou les immunoglobulines qui en sont extraites) de protéger ne fût-ce que temporairement seulement un animal non immunisé, le cas échéant spénectomisé, et sensible contre le parasite, notamment la souche FUPC I-212, lorsque ce sérum est passivement transféré in vivo de l'animal immunisé à l'animal non immunisé.

En particulier, on pourra utiliser comme source d'anticorps protecteurs pour la reconnaissance des fractions polypeptidiques conformes à l'invention les sérums, ascites ou fractions immunoglobuliniques obtenues à partir de singes et qui sont capables, par transfert passif in vivo à des singes receveurs splénectomisés sensibles, de les protéger contre l'infection parasitaire, lorsque ceux-ci ont reçu au préalable, par voie intraveineuse, une injection de 50 x $10^6$ cellules parasitées, elles-mêmes obtenues à partir de singe splénectomisé et infecté et prélevées au moment où l'infection était aiguë et en phase ascendante. De préférence, on aura recours à des sérums, ascites ou fractions immunoglobuliniques qui permettent cette protection, lorsqu' ils sont administrés à l'animal sensible à des doses telles que la teneur du sang en immunoglobulines reçues par l'animal receveur ne dépasse pas 1 mg par ml. Chez Saimiri Sciureus, on peut utiliser à titre de source d'anticorps protecteurs les immunoglobulines qui permettent la susdite protection, lorsqu'ils sont administrés - deux à trois jours après l'infection - à l'animal receveur par voie intrapéritonéale, à raison de doses quotidiennes de 3 à 10 mg d'immunoglobulines, ou par voie intraveineuse, à raison de doses quotidiennes de 0,5 à 2 mg d'immunoglobulines, pendant une durée de 3 à 6 jours. L'effet protecteur est lui-même évalué par l'inhibition et le contrôle alors observé de la parasitémie pendant et après le traitement, par comptage (sous le microscope) des pourcentages de globules rouges parasités dans des frottis de sang coloré par le Giemsa.

Les parasites utilisés pour l'invention peuvent être constitués par la souche de P . falciparum FUPC I-212 susdite.

Les animaux immunisés, en particulier les singes Saimiri Sciureus, sont des animaux qui ont pu être eux-mêmes exposés aux parasites infectieux et traités soit par chimiothérapie, par exemple la quinine, soit par administration d'immunoglobulines protectrices provenant d'un autre animal, lui-même immunisé. D'une façon générale, ces anticorps protecteurs sont présents dans des animaux en état d'infection ou de sub-infection chronique. Il est à cet égard significatif que les anticorps protecteurs peuvent n'être pas présents en quantités significative à la fin de la période d'infection aiguë chez l'animal receveur, bien qu'à ce moment-là des titres élevés d'anticorps anti-malariques peuvent être détectés in vitro par des techniques classiques d'immunofluorescence. Ces anticorps protecteurs sont par contre susceptibles d'apparaître dans un délai de 30 à 90 jours, à compter de la fin de l'infection aiguë.

De plus, la présence d'anticorps protecteurs n'est souvent pas corrélable avec la teneur du sérum en anticorps ayant une activité inhibitrice in vitro .En particulier, on constate que des préparations d'immunoglobulines peuvent, par transfert passif in vivo , dans les conditions qui ont été indiquées, manifester une activité protectrice importante, tout en étant dépourvues d'effet inhibiteur in vitro contre le parasite en culture dans des globules rouges humains ou du singe Saimiri Sciureus.

La source d'immunoglobulines utilisable pour la détection des polypeptides selon l'invention soit est constituée par un sérum entier contenant lesdits anticorps immunoprotecteurs, soit par des ascites préalablement provoqués chez l'animal protégé, soit encore par des fractions d'immunoglobulines obtenues à partir du sérum ou de l'ascite. Les conditions dans lesquelles les ascites peuvent être formés sont connues. On rappelle pour mémoire que ceux-ci peuvent par exemple être formés par injection intrapéritonéale d'adjuvant de Freund émulsionné dans une solution saline appropriée. Des immunoglobulines davantage purifiées peuvent être obtenues à partir du sérum ou de l'ascite, de façon en soi connue également, par exemple par passage du fluide sur un support, par exemple celui commercialisé sous la désignation "SEPHAROSE 4B", sur lequel au préalable a été fixée de la protéine A. La fixation des immunoglobulines peut être effectuée en présence d'un tampon phosphate pH 7,4. Les immunoglobulines fixées peuvent ensuite, après lavage poussé de la colonne avec un tampon adéquat, par exemple le tampon PBS, être éluées avec de l'acide acétique 1M, puis neutralisées, dialysées contre du PBS et avantageusement concentrées jusqu'à atteindre un volume du même ordre de grandeur que le volume de sérum ou d'ascite initialement mis en oeuvre.

Une caractéristique supplémentaire des anticorps protecteurs intervenant dans la caractérisation des fractions polypeptidiques selon l'invention réside dans leur capacité à reconnaître non seulement des polypeptides de poids moléculaire élevé, par exemple de l'ordre de 200.000, parmi les produits de dissolution du parasite, mais également des fractions polypeptidiques de plus faible poids moléculaire conformes à l'invention (ayant notamment des poids molélculaires s'étageant entre 75.000 et 100.000). En cela ils se distinguent d'anticorps non protecteurs qui reconnaissent les poids moléculaires élevés et ne reconnaissent pas les poids moléculaires 75.000 et 100.000.

L'invention concerne encore plus particulièrement des polypeptides "intacts" en ce sens qu'ils ne consistent pas en des fragments de protéines membranaires ayant initialement eu des poids moléculaires plus élevés, synthétisés au stade des schizontes et ayant ensuite subi une digestion intracellulaire. En particulier, des polypeptides immunologiquement identiques à des polypeptides préférés de l'invention ( ayant un poids moléculaire de l'ordre de 76.000 ) peuvent être synthétisés dans un système non cellulaire ou "exempt de cellules" (cell free system), notamment dans un lysat de réticulocytes de lapin, par traduction in vitro d'ARNs messagers extraits du parasite au stade schizonte, dans des conditions où les protéines synthétisées sont rendues résistantes à la digestion tryptique. Avantageusement la traduction est réalisée en présence de microsomes de rein de chien, ajoutés au préalable au lysat.Les mêmes observations peuvent être faites avec les autres polypeptides capables de réagir avec des anticorps protecteurs et présentant des poids moléculaires analogues moyen à ceux qui ont été mentionnés plus haut, et en particulier des polypeptides ayant des poids moléculaires de 72.000, 76.000, 90.000, 100.000 et 110.000. Ces observations suggèrent donc que les ARNs messagers mis en oeuvre en contenaient certains qui étaient spécifiques de ces polypeptides, de sorte que ceux-ci ne résultaient pas, même à l'occasion de la traduction intracellulaire des ARNs messagers correspondants, de la dégradation de protéines possédant en fait des poids moléculaire plus élevés.

L'invention concerne encore un procédé d'obtention de tels polypeptides protecteurs, comportant les étapes consistant à traiter une préparation préalablement obtenue d'un parasite malarique infectieux, notamment du type plasmodium, tel que Plasmodium falciparum avec une solution d'un détergent, tel que le Sodium Dodecyl Sulfate (SDS) ou celui connu sous la désignation commerciale TRITON x 100, ou analogue, susceptible d'induire la dissolution de la majeure partie des structures cellulaires et des constituants protéiniques du parasite, à séparer et à récupérer à partir de la solution formée ceux des polypeptides qui présentent les susdits poids moléculaires moyens s'étageant d'environ 50000 à environ 140.000 ou contenus dans les intervalles de poids moléculaires moyens qui ont été indiqués plus haut et qui contiennent des polypeptides immunogènes et capables à la fois d'induire la production d'anticorps protecteurs contre des parasites infectieux contre l'homme et/ou le singe et d'être reconnus par des anticorps protecteurs obtenus à partir de singes immunisés. De préférence, le traitement susdit de dissolution est effectué en présence d'un inhibiteur de protéases.

Une purification supplémentaire peut être obtenue par réaction avec ces anticorps protecteurs ou équivalents, préalablement fixés sur un support insoluble. Par "anticorps équivalents, on entend ici des anticorps préalablement formés et capables de réagir avec les mêmes peptides immunogènes. Il s'agit par exemple d'anticorps monoclonaux obtenus à partir d'hybridomes résultant de fusions cellulaires ayant mis en jeu des souris immunisées contre des fractions extraites de P . falciparum et correspondant à l'un des poids moléculaires susindiqués. On peut ensuite recueillir les polypeptides immunogènes ayant formé un complexe avec l'anticorps utilisé, par exemple en ayant recours à une technique semblable à celle qui a été indiquée plus haut, lorsqu'a été décrit un exemple d'enrichissement des immunoglobulines contenues dans un sérum ou ascite d'un animal immunisé contre les parasites.

Avantageusement, il s'agit d'anticorps obtenus à partir d'hybridomes formés à partir de cellules de souris et immunisées contre l'un des polypeptides capables à la fois d'induire la production d'anticorps producteurs contre des parasites infectieux et d'être retenus par les anticorps producteurs obtenus à partir de singes immunisés. Un anticorps monoclonal préféré pour réaliser la purification de l'un des peptides conformes à l'invention est constitué par l'anticorps déposé à la C.N.C.M. sous le n° I-271.

En variante, on peut également obtenir des fractions immunogènes conformes à l'invention à partir de la susdite solution des structures cellulaires et des constituants protéiniques du parasite, par mise en contact direct de cette solution avec un anticorps fixé, tel que sus-défini, puis dissocier le complexe formé en vue de récupérer les antigènes fixés et, le cas échéant, séparer et récupérer les différents polypeptides immunogènes présents, selon leurs poids moléculaires respectifs.

L'invention n'est pas limitée aux susdites fractions. Elle s'étend encore aux fractions polypeptidiques qui peuvent être obtenues à partir de parasites qui peuvent être considérés comme des dérivés ou même encore des mutants de Plasmodium falciparum . Notamment sont inclus dans le cadre des revendications de cette demande tous les polypeptides immunogènes, qui sont reconnus par des anticorps protecteurs, obtenus à partir d'animaux résistants à la souche de Plasmodium falciparum déposée à la C.N.C.M. sous le n° FUPC I-212 le 23 décembre 1982, et plus particulièrement encore les anticorps protecteurs plus spécifiques susceptibles d'être obtenus à partir des animaux immunisés avec les polypeptides protecteurs selon l'invention issus de cette même souche. Sont également inclus dans le cadre de la présente demande les polypeptides obtenus à partir de parasites malariques infectieux, quelle que soit leur origine, qui sont à la fois protecteurs et reconnus par des anticorps monoclonaux secrétés par les hybridomes formés dans les conditions qui ont également été indiquées, en mettant en oeuvre, pour l'immunisation des animaux donneurs des cellules spléniques requises, les polypeptides conformes à l'invention et obtenus à partir de la souche FUPC I-212.

Il en est particulièrement ainsi des polypeptides issus de souches autres que P . falciparum, et qui sont reconnus par les anticorps de singe protecteurs et éventuellement par l'anticorps monoclonal secrété par l'hybridome I-271 identifié plus haut. A titre d'exemple de "sources" de polypeptides conformes à l'invention, on mentionne P . vivax , P . ovale , P . chabaudi , P . yoelli , P . knowlesi , etc..

D'une façon générale, on pourra, à partir d'une préparation donnée de parasites, déterminer ceux des constituants protéiniques susceptibles d'induire in vivo la production d'anticorps protecteurs en procédant comme suit.

On part d'une culture du parasite étudié, préalablement réalisée au sein d'un milieu de culture contenant un marqueur radioactif spécifique des constituants protéiniques des parasites en question, tel que la méthionine $^{35}$S. Les constituants parasitaires sont alors recueillis et traités comme il a été décrit plus haut en ce qui concerne Plasmodium falciparum ou de façon plus détaillée dans les exemples qui suivent, mais en vue de dissoudre la majeure partie des constituants cellulaires et protéiniques du parasite choisi, sauf que l'on utilise le détergent TRITON X100 en lieu et place du SDS. Le TRITON X100 solubilise en effet les protéines antigéniques sans altérer profondément leur structure, de sorte qu'elles peuvent toujours encore être reconnues par des anticorps- On procède alors aux séries d'opérations suivantes. Première série d'opérations a) On fait une électrophorèse d'un échantillon de l'ensemble des constituants protéiniques des parasites. On obtient toute une série de bandes visualisables par autoradiographie. Par comparaison avec des protéines dont les poids moléculaires sont connus, on détermine les successions de poids moléculaires, par rapport aux migrations réalisées de façon concomitante par des peptides de poids moléculaires connus. b) Partant d'un échantillon distinct desdits constituants protéiniques (il s'agit toujours de la préparation marquée), on le fait réagir avec un sérum contenant des anticorps protecteurs et provenant d'un singe résistant au parasite on fait ensuite réagir l'ensemble avec la protéine A du staphylocoque doré (préparation commerciale). On sépare par centrifugation le précipité formé, qui contient un complexe protéine A - anticorps - antigène radioactif. Après lavage du précipité, on le redissout en présence de SDS. Celui-ci dissocie l'antigène de l'anticorps et de la protéine A. On resoumet l'ensemble à électrophorèse et on repère les bandes marquées, en nombre plus réduit que dans le cas précédent, ainsi que leurs poids moléculaires respectifs.

Deuxième série d'opérations :

On répète l'ensemble des étapes a) et b) ci-dessus, mais en utilisant cette fois-ci des anticorps provenant d'un singe sensible au parasite.

Par comparaison des bandes obtenues sur les gels après les réactions avec les sérums provenant d'animaux résistants, d'une part, et de celles obtenues après réaction avec des sérums provenant d'animaux sensibles, d'autre part, on détermine celles des bandes qui n'ont été obtenues qu'au terme de l'étape b) de la première série d'opérations et leurs régions de poids moléculaires.

Ces bandes contiennent des constituants protéiniques reconnus préférentiellement par des sérums d'animaux résistants et, de ce fait, les antigènes reconnus spécifiquement par les anticorps protecteurs.

Troisième série d'opérations :

On réalise enfin une nouvelle culture du même parasite, mais en l'absence du marqueur radioactif, et on procède à une nouvelle séparation électrophorétique de ses constituants protéiniques dans les mêmes conditions que dans la première et deuxième série d'opérations. On teste ensuite la capacité des constituants ayant migré aux mêmes distances que ceux qui ont été reconnus par les anticorps à l'issue de la deuxième série d'opérations, à induire in vivo la production d'anticorps protecteurs, et l'on recueille ceux de ces constituants qui ont induit une réaction immunologique positive, par exemple dans les mêmes conditions que celles évoquées précédemment à propos des constituants protéiniques actifs de

Plasmodium falciparum .

Les constituants actifs ainsi isolés constituent des équivalents des polypeptides protecteurs qui ont plus particulièrement été décrits plus haut.

Des caractéristiques supplémentaires de l'invention apparaîtront encore au cours de la description qui suit d' exemples de production de fractions polypeptidiques selon l'invention, à partir d'une culture de parasites, et des propriétés biologiques de ces fractions.

EXEMPLE I

Culture des parasites et préparation.

Les parasites sont cultivés sur globules rouges humains de groupe A⊕, dans du milieu RPMI 1640 (FLOBIO) additionné de 10% de sérum humain du même groupe, sous une atmosphère contenant 1 % O₂, 3 % CO₂, 96 % N₂ (% en volumes)

Les cultures sont synchronisées au stade "anneaux", tel que défini dans l'article de Science 1976, 193 , 673, par traitement au sorbitol (LAMBROS 1979, vol. 65 , P. 418 J. of Parasitology). Elles sont récoltées 40 heures plus tard, lorsque les parasites atteignent le stade de schizontes murs, à 8 noyaux en moyenne.

Les globules rouges sont lavés par centrifugation en RPMI 1640 et lysés par action de la Saponine à 0,025 % en RPMI (% en poids), en présence d'inhibiteurs de protéases "PMSF" , "TLCK" (SIGMA) $10^{-3}$ M, pendant 10 minutes à 37° C.

Les parasites libres sont purifiés par centrifugation dans un gradient discontinu en PBS comportant 40 à 70 % de PERCOLL (PHARMACIA), pendant 30 minutes à 4°C. L'interface 40-70 % est récoltée par pipettage et lavée deux à trois fois en PBS.

Préparation des protéines semi-purifiées .

Les parasites sont traités par 5 à 10 volumes de tampon pour électrophorèse, contenant 6 % en poids de SDS et 10 % de bêta-mercapto-éthanol (ou d'un agent analogue ssuceptible de couper les ponts disulfure des protéines et de linéariser les chaînes polypeptidiques) deux fois pendant 5 minutes à 100°C.

Les extraits sont centrifugés 5 minutes à 15.000 g, les surnageants (contenant plus de 90 % des protéines totales initialement contenues dans les parasites) sont soumis à une électrophorèse (pendant 4 heures sous 200 V) en gel contenant 10 % en poids/volume d'acrylamide au sein d'un tampon Tris aqueux (à pH 8-9 ) contenant 0,2 % de SDS (en poids/volume).

Après électrophorèse, des bandes de gels sont coupées aux niveaux correspondants aux poids moléculaires 75.000 + 7.500 daltons repérés sur le gel par électrophorèse simultanée de peptides marqués à l'isothyocyanate de fluorescéine (IgG humaines, BSA, ovalbumine, phosphorylase B et la myosine ).On découpe de la même façon les bandes de gels contenant les polypeptides présentant des poids moléculaires moyens de 100.000 + 10.000, récoltés dans un sac à dialyse. La concentration en protéines est déterminée par dosage colorimétrique des protéines ("Biorad Assay", kit commercialisé par BIORAD et décrit par SPECTOR, Analytical Biochemistry, 1978, 86 , 142).

Les préparations finalement obtenues sont analysées par électrophorèse en gel polyacrylamide-SDS et révélées par coloration au nitrate d'argent. On peut ainsi encore séparer à partir de la préparation de polypeptides ayant des poids moléculaires de 75.000 + 5.000 :

- 3 bandes majeures dans la zone de poids moléculaires : 72.000, 76.000, 80.000 daltons,
- 1 bande mineure dans la zone de poids moléculaires : 90.000 daltons.

Des produits de dégradation mineurs de cette bande de 75.000 sont vus dans les zones 33.000 et 45.000 daltons.

De même, on peut encore séparer dans les mêmes conditions, à partir de la fraction de poids moléculaires 100.000 + 10.000, des bandes majeures isolées dans les zones suivantes de poids moléculaires :
40.000, 45.000, 100.000, 110.000, 115.000 et 130.000, parmi un ensemble complexe étendu, s'étageant entre 83.000 et 140.000. Des produits de dégradation mineurs de cette bande sont vues dans les zones 33.000 et 45.000 daltons.

Les propriétés biologiques de cette fraction, qu'il s'agisse des fractions de poids moléculaires moyens 75.000 ou 100.000, ou des fractions protéiniques davantage purifiées, qui peuvent être isolées à partir des précédentes, peuvent être mises en évidence comme décrit ci-après. Les résultats qui suivent ont été obtenus avec les fractions 75.000 et 100.000, avant purification supplémentaire.

Essai d'immunisation de singes Saimiri Sciureus .

Les résultats d'immunisation susceptibles d'être observés dans ces primates sont particulièrement représentatifs de ce qui pourrait être obtenu de façon semblable chez l'homme. Ces animaux peuvent être davantage encore rendus sensibles à l'infection parasitaires par splénectomie : l'infection se développe rapidement chez des singes splénectomisés. Elle se manifeste par des parasitémies importantes (50 % environ de leurs globules rouges pouvant être parasités). Les animaux splénectomisés et impaludés meurent au bout d'une durée de l'ordre de 10 jours.

Les essais qui suivent ont été réalisés chez des animaux splénectomisés répartis respectivement en deux groupes de 5 et 10 singes.

Les animaux du premier groupe sont inoculés trois fois avec chaque fois une dose de 100 microgrammes des préparations à tester diluées dans 0,5 ml de PBS émulsionné avec un volume égal d'adjuvant de Freund, complet pour la première inoculation, incomplet pour les deux suivantes. Ces inoculations se font à trois semaines d'intervalle, sous la forme d'injections sous-cutanées.

Les singes du deuxième groupe (témoins) ne reçoivent que les adjuvants.

Trois semaines après la troisième immunisation, les animaux sont infectés avec $50 \times 10^6$ globules rouges parasités, par voie intraveineuse. La parasitémie est ensuite contrôlée chaque jour pendant trois semaines. On observe tout de suite un important retard de la multiplication parasitaire chez ceux des animaux qui ont été immunisés. 15 jours plus tard, la proportion de globules rouges parasités chez les animaux immunisés est inférieure à 10 %, alors que chez le groupe témoin -5jours après la dernière injection - la parasitémie est de plus de 25 %. On a obtenu des résultats semblables avec les deux préparations.

Ces résultats sont hautement significatifs, compte tenu du caractère splénectomisé des animaux. Des résultats semblables peuvent être obtenus avec des fractions davantage purifiées encore.

EXEMPLE II.

Culture des parasites et préparation .

Les parasites (provenant de la souche de P . falciparum , C.N.C.M. n° FUPC I-212) ont été cultivés sur globules rouges humains A⁺, selon la technique décrite par GYSIN J. et Coll. (Les Ann. Immunol. (Institut Pasteur) 133 D, 95-102 (1982)) et synchronisée par la technique au sorbitol telle que décrite par LAMBROS C. et Coll. (J. Parasitol. 65 , 418-420 (1979)).

Les cultures infectées ont été collectées quand la parasitémie a atteint 5 à 10 % des cellules et quand les parasites ont atteint le stade des schizontes. Après deux lavages avec du milieu RPMI-1640 (GIBCO), les globules rouges ont été lysés par la saponine (à 0,025 % en poids) pendant 10 minutes à 37°C. La suspension a ensuite été refroidie à 4°C et centrifugée à 3.500 9 pendant 10 minutes. Le culot a été resuspendu dans du RPMI-1640, introduit dans un gradient au PERCOLL (produit fabriqué par PHARMACIA) (20-40 %), puis centrifugé à 3.500 g à 4°C pendant 30 minutes. Les parasites libres ont été collectés à l'interface 20/40 lavés deux fois dans le tampon PBS et congelés à -20°C, en présence d'inhibiteurs de protéase, tels que ceux commercialisés sous les désignations TLcK et PMSF (SIGMA).

Préparation des protéines immunogènes semi-purifiées .

Les préparations de parasites (20/40) sont introduites dans 5 volumes d'une solution tampon (tris 0,0625 M, pH 6,8, dodécylsulfate de sodium à 6 %, 2-mercaptoéthanol à 5 %, glycérol à 5 %) portés à l'ébullition pendant 5 minutes et centrifugés à 15.000 g pendant 10 minutes.

Le surnageant a été appliqué sur un gel de SDS-polyacrylamide, en parallèle avec des marqueurs de poids moléculaires déterminés et marqués à l'isothiocyanate de fluorescéine (sérum albumine bovine, IgG de lapin et ovalbumine B-phosphorylase et myosine. Après migration à travers le gel, les bandes de poids moléculaires correspondant aux zones 70.000 - 85.000 et 90.000 - 120.000 respectivement (vis-à-vis des distances de migration des marqueurs) ont été découpées. Les protéines ont ensuite été éluées à partir de ces bandes de gel par électrophorèse dans un tampon trisglycine, pH 8,6, et contenant 0,1 % en poids de dodécylsulfate de sodium (SDS), et récoltées dans un sac à dialyse. La concentration en protéines a été déterminée par dosage colorimétrique des protéines, méthode au bleu de Coomasie, "Biorad Assay", test commercialisé par BIORAD et décrit par Spector, "Analytical Biochemistry", 1978, 86 , 142.

On a ainsi obtenu les fractions I et II ultérieurement utilisées dans les essais d'immunisation décrits ci-après. Ces fractions contenaient environ 100 microgrammes de protéine/20 mg de protéine contenue dans l'extrait brut initial.

Ces fractions se sont révélées contenir des bandes de poids moléculaires distincts, à l'occasion d'un refractionnement dans un gel de polyacrylamide - SDS à 7,5 %. Ces fractions ont été révélées par coloration par le nitrate d'argent. La fraction I s'est révélée contenir quatre bandes polypeptidiques majeures de poids moléculaires 72.000, 76.000 85.000 et 90.000 et la fraction II s'est révélée contenir principalement cinq bandes de poids moléculaires moyens 90.000, 96.000, 100.000, 105.000, et 120.000, respectivement.

La présence de bandes mineures correspondant à la région 50.000 daltons a été détectée dans les deux préparations.

Essai d'immunisation de singes écureuils .

Des groupes de cinq animaux splénectomisés reçoivent trois injections sous-cutanées.successives aux jours 0, 21 et 4l. Chaque injection consiste en 100 microgrammes soit de la fraction I, soit de la fraction II, contenue dans une émulsion formée à partir d'une solution saline tamponnée au phosphate et contenant 0,1 % de SDS et d'un même volume de l'adjuvant complet de Freund (pour les premières injections) ou de l'adjuvant incomplet de Freund (pour les injections suivantes). Dix animaux témoins ne reçoivent que la solution saline tamponnée au phosphate (PBS) contenant 0,1 % de SDS avec l'adjuvant complet ou l'adjuvant incomplet de Freund pour l'applicaion du même protocole d'administration. Des prélèvements de sang sont faits périodiquement, avant et pendant la vaccination, et examinés pour ce qui est de leurs caractéristiques hématologiques,parasitologiques, microbiologiques et sérologiques. Les animaux ont très bien toléré la vaccination. Aucune lésion n'a été observée aux sites d'injection. Les singes ont toujours paru en bonne santé et manifestaient une activité normale. Aucune anémie n'a été détectée tant chez les animaux vaccinés que chez les animaux témoins.

Des anticorps antimalariques, mesurés par immunofluorescence indirecte, ont été détectés dès après la seconde injection.

Les animaux ont reçu, par injection intraveineuse, 50 x 10⁶ parasites (souche FUP) au jour 55. Après cette épreuve, les parasitémies ont été mesurées quotidiennement sur des prélèvements colorés par le réactif de Giemsa. La parasitémie aiguë qui s'est développée chez neuf animaux sur les dix animaux témoins a nécessité une chimiothérapie à la quinine dans les huit jours qui ont suivi, pour éviter la mort. Parmi les cinq animaux ayant reçu la fraction I, un seul a présenté une réponse semblable à celle des témoins. Les quatre autres animaux ont manifesté une résistance élevée à l'épreuve. Une parasitémie marginale a été observée dans deux d'entre eux. Dans les deux autres, la parasitémie s'est élevée à environ 5 % pour disparaître complètement dans les trois semaines suivantes. Les cinq animaux immunisés avec la fraction II ont tous manifesté une bonne résistance à l'épreuve. On a observé un accroissement de 10 % de la parasitémie au dixième jour après l'épreuve chez l'un d'entre eux seulement. Cette parasitémie a cependant décru dans les trois semaines suivantes.

Des frottis de contrôle ont été ensuite effectués deux fois par semaine pendant les deux mois qui ont suivi l'épreuve. Les mesures ont donné des résultats négatifs dans tous les animaux vaccinés, alors qu'une recrudescence de la parasitémie a été observée dans cinq des animaux témoins qui avaient été traités par la quinine, après l'interruption du traitement, de sorte qu'ils ont dû être soumis à un second cycle de chimiothérapie. Tous les animaux infectés par le parasite (animaux vaccinés et animaux témoins) ont présenté dans les premières semaines une certaine anémie avec une chute moyenne de 30 % des taux en globules rouges. L'anémie a cependant été différée dans les animaux vaccinés. En outre les hématocrites ont repris une valeur normale quatre semaines après l'épreuve. Les variations de poids des animaux avant et après la vaccination n'ont pas dépassé 2 %.

La réponse humorale des singes aux différentes immunisations a été appréciée par immunoprécipitation réalisée entre des extraits de parasites marqués à la $^{35}$S-méthionine et des échantillons de sérum prélevés chez les animaux avant et après les susdites immunisations. On n'a observé aucune immunoprécipitation spécifique avec les échantillons de sérum prélevés chez tous les animaux avant l'immunisation et chez les animaux témoins, avant l'épreuve par le parasite vivant. Chez les animaux immunisés aussi bien par la fraction I que par la fraction II, on a constaté des réponses sensiblement homogènes contre principalement deux polypeptides de masse moléculaire 72 et 90.000 et des réponses de moindre importance à l'égard de peptides de masse moléculaire 96.000 et 100.000.

Une bande de précipitine dans la région de poids moléculaire 110.000 a été constatée chez les animaux vaccinés avec la fraction II. Aucune réponse n'a été constatée, du moins dans les conditions de l'expérience, contre la bande 76.000 contenue dans la fraction I originale. Cette dernière observation trouve peut-être son explication dans la modification des déterminants antigéniques que présente la protéine répondant à ce poids moléculaire chez les parasites vivants au cours des étapes de purification décrites plus haut. Les réponses comparables qui ont été observées dans les deux groupes d'animaux immunisés donnent à penser qu'il existe une parenté anti-génique importante entre les protéines des deux fractions, parenté qui explique sans doute le degré de résistance comparable contre le parasite, qui a été observé dans les deux groupes.

Les résultats qui précèdent témoignent donc de l'importante capacité vaccinante des fractions de l'invention. Ce résultat est d'autant plus remarquable, que cette vaccination a été obtenue avec des protéines de parasites "dénaturées" par le SDS. La dénaturation chimique des protéines par le détergent n'entraîne pas la perte des propriétés vaccinantes de ces dernières.

Les fractions peptidiques selon l'invention constituent tout d'abord des réactifs biologiques particulièrement intéressants en ce qu'ils sont actifs in vivo . Ils peuvent être utilisés comme référence de mesure de l'activité in vivo d'autres préparations immunogènes obtenues à partir de parasites responsables des diverses formes de paludisme.

Une purification plus poussée de l'antigène vaccinant contenu dans les susdites fractions peut être réalisée selon les techniques décrites plus **haut.** L'invention concerne plus particulièrement les fractions de poids moléculaires 72.000 et 90.000. Les fractions de l'invention davantage purifiées ou non, y inclus la fraction de poids moléculaire 50.000, peuvent également être utilisées comme réactifs pour le diagnostic et/ou le titrage in vitro des anticorps antipaludéens. Dans leur utilisation comme réactifs pour un diagnostic in vitro , on peut avoir recours à des techniques classiques, par exemple à la technique ELISA. On rappelle ci-après le principe d'une telle méthode. Elle comprend, par exemple, les étapes suivantes :

- dépôt de quantités déterminées d'un polypeptide selon l'invention dans les puits d'une microplaque du type de celle utilisée pour la mise en oeuvre de la méthode ELISA ;
- introduction de dilutions croissantes du sérum contenant, le cas échéant, les anticorps à détecter, voire à doser, dans les puits de cette microplaque ;
- incubation de la microplaque et de son contenu ;
- lavage poussé de la microplaque avec un tampon approprié;
- introduction d'anticorps marqués dirigés contre les premiers, le marquage étant fait au moyen d'une molécule fluorescente ou d'une enzyme capable d'hydrolyser un substrat choisi parmi ceux pour lesquels cette hydrolyse se manifeste par une variation d'absorbance pour une radiation de longueur d'onde donnée ;

- mesure de l'intensité de fluorescence ou de la variation d'absorbance et
- détermination, de préférence vis-à-vis de mesures semblables faites vis-à-vis d'un témoin, de la teneur en anticorps du sérum étudié.

L'invention concerne encore plus particulièrement un nécessaire ou coffret de diagnostic in vitro du paludisme contenant plus particulièrement:

- l'un des polypeptides définis dans ce qui précède, ce polypeptide étant marqué par une enzyme ou par un composé fluorescent ;
- d'anticorps spécifiques du polypeptide utilisé,
- des tampons et substrats éventuellement nécessaires à la révélation du marqueur.

Il va naturellement de soi que tout autre marqueur peut être utilisé, par exemple un marqueur radioactif.

Le coffret peut également permettre de suivre chez le malade l'évolution de la maladie par dosage des anticorps et des parasites présents dans le sang du sujet atteint.

Il convient de souligner que l'antigène de poids moléculaire 72.000 est immunoprécipité avec des immunsérums anti-P . vivax , anti-P . ovale , anti-P . chabaudi . Cet antigène apparaît comme un constituant commun à différentes espèces de Plasmodium .

A titre d'exemple, on a incubé un extrait polypeptidique de P . chabaudi marqué par $^{35}$S-méthionine (300.000 cpm) avec 10 microlitres d'immunsérum anti-P. falciparum obtenu après immunisation de Saimiri Sciureus ou d'un patient résistant à P . falciparum vivant en zone endémique ou encore de souris immunisées avec la fraction selon l'invention. Sur gel, l'analyse des bandes immunoprécipitées, révèle cet antigène de 72.000 daltons produit par P . chabaudi .

Une observation identique a été faite avec la fraction de poids moléculaire 90.000 préparée à partir de P . chabaudi .

Ces résultats sont particulièrement intéressants, dans la mesure où ils montrent que l'antigénicité des peptides obtenus à partir de ces différents Plasmodiums n'a pas une spécificité d'espèce. On remarque en particulier que P . chabaudi est un Plasmodium se développant chez la souris. Il en résulte que l'utilisation des polypeptides antigéniques issus de P . chabaudi ou de tout autre Plasmodium se développant dans des petits animaux, sera particulièrement avantageuse, en ce sens que la culture de ces Plasmodiums est moins onéreuse que celle de P . falcipa rum dont l'un des hôtes naturels est le grand singe.

L'invention concerne encore plus particulièrement les compositions vaccinantes contenant lesdites fractions polypeptidiques en association, comme on l'a déjà indiqué, avec les divers véhicules pharmaceutiques et/ou agents adjuvants couramment utilisés dans les compositions de vaccins. De préférence, l'invention concerne des solutions injectables desdites fractions polypeptidiques, par exemple pour l'administration intraveineuse ou intramusculaire. Les compositions contiennent avantageusement, de 0,5 à 10 mg, notamment de 1 à 5 mg d'immunoglobulines par dose à administrer.

Les fractions polypeptidiques selon l'invention peuvent encore être utilisées pour la fabrication d'anticorps plus spécifiques à l'égard de Plasmodium falciparum que les sérums ou fractions purifiés d'immunoglobulines susceptibles d'être obtenus à partir d'animaux devenus résistants. Avantageusement, on aura recours pour la fabrication de ces anticorps plus spécifiques à la technique de fabrication des hybrides cellulaires ou hybridomes,comportant alors les étapes essentielles suivantes (susceptibles d'être mises en oeuvre selon des techniques aujourd'hui connues ) :

- fusion de cellules myélomateuses et de cellules spléniques de souris ou de rat, voire de singe, ayant au préalable été immunisées avec les fractions peptidiques sus-définies,
- sélection parmi les hybridomes formés de ceux qui sécrètent des anticorps monoclonaux actifs contre les susdites fractions polypeptidiques.

Ces anticorps plus spécifiques, particulièrement ces anticorps monoclonaux, peuvent alors être à leur tour utilisés, par exemple pour constituer des colonnes de chromatographie d'affinité. Une telle colonne est par exemple obtenue par fixation de ces anticorps monoclonaux sur la résine commercialisée par PHARMACIA sous la marque SEPHAROSE par la méthode bien connue au bromure de cyanogène. Ces colonnes de chromatographie d'affinité peuvent alors à leur tour être utilisées pour réaliser la séparation de fractions polypeptidiques immunogènes, que ce soit à partir de solutions obtenues à partir de préparations de parasites traités avec des agents dissolvants, du genre de ceux qui ont été mentionnés plus haut, ou à partir de fractions déjà concentrées et dont un état de plus grande pureté est recherché.

Comme il va de soi et comme il résulte d'ailleurs déjà de ce qui précède, l'invention s'étend aussi à toutes les variantes et à toutes les compositions possédant des propriétés immunologiques semblables. En particulier, l'invention concerne encore toutes les fractions peptidiques immunogènes susceptibles d'être obtenues à partir d'autres Plasmodiums , notamment P . vivax , P . ovale et P . chabaudi , P . yaelli et P . knowlesi déjà nommés, etc., en mettant en oeuvre des procédés semblables à ceux qui viennent d'être décrits. D'une façon générale, on pourra utiliser comme source des polypeptides immunogènes selon l'invention tout Plasmodium , que celui-ci soit infectieux pour l'homme ou pour l'animal seulement.

D'une façon générale, l'invention concerne des polypeptides immunogènes susceptibles d'être obtenus à partir de tout Plasmodium , de tous variants et mutants de ces Plasmodiae , dès lors que ces polypeptides sont reconnus par des anticorps formés plus particulièrement contre les fractions qui ont été définies plus haut. L'invention concerne également encore des peptides immunogènes de poids moléculaire plus faible, par exemple des peptides immunogènes résultant d'une hydrolyse partielle des peptides précédents, dans la mesure où cette hydrolyse ne porte pas atteinte à l'immunogénicité recherchée. Entrent encore dans le cadre de l'invention tous conjugués entre l'un des peptides immunogènes envisagés ci-dessus et une molécule porteuse ("carrier"), chaque fois qu'un tel couplage peut être jugé désirable pour renforcer l'immunogénicité des peptides en cause.

D'une façon générale, l'invention concerne donc tous polypeptides répondant aux conditions qui ont été sus-définies et qui pourraient être reconnus par des anticorps monoclonaux préalablement formés contre chacun des polypeptides correspondant aux poids moléculaires moyens qui ont été indiqués, notamment 75.000 et 100.000, ou correspondant encore à des fractions purifiées dont les constituants majeurs sont respectivement formés de polypeptides ayant des poids moléculaires de l'ordre de 72.000, 75.000 (ou 76.000), 85.000, 90.000, 96.000, 100.000, 105.000 et 120.000. Il résulte naturellement de ce qui précède que ces hybridomes peuvent être formés par la méthode devenue classique de Köhler et Milstein. La sélection finale des hybridomes secréteurs des anticorps monoclonaux recherchés est alors notamment basée sur la détection des anticorps qui reconnaissent des polypeptides ayant les poids moléculaires correspondant à ceux ayant initialement servi à la production des hybridomes correspondants et qui sont également reconnus par les anticorps protecteurs obtenus à partir de singes devenus résistants au parasite de la malaria ou encore par le sérum humain provenant de personnes immunisées résidant dans des zones endémiques et présentant un pouvoir de résistance élevé aux Plasmodiae .

## Revendications

1. composition immunogène, contenant un ou plusieurs polypeptides choisis parmi des polypeptides ayant des poids moléculaires de l'ordre de 50.000 à 140.000, ces polypeptides étant caractérisés :
   - en ce qu'ils sont reconnus par des anticorps protecteurs contre les parasites du paludisme, ces anticorps étant contenus dans des sérums, ascites ou autres compositions d'immmunoglobulines, provenant de singes Saimiri Sciureus rendus résistants à la souche de P . falciparum déposée à la C.N.C.M. sous le numéro I-212, cette résistance étant obtenue par infection de ces singes par ladite souche de P.falciparum (devenue virulente par passages successifs de ladite souche chez plusieurs singes Saimiri Sciureus splenectomisés) suivie d'un traitement par la quinine, ces sérums, ascites ou autres compositions d'immunoglobulines, étant capables par transfert passif in vivo à des singes receveurs splénectomisés sensibles, de les protéger contre l'infection par ladite souche de P. falciparum , lorsque ceux-ci ont reçu au préalable l'injection de 50.10⁶ cellules parasitées par ladite souche de P. falciparum, ces cellules étant elles-même obtenues à partir de singes Saimiri Sciureus splénectomisés et infectés par ladite souche de P. falciparum, et au moment où l'infection était aiguë et en phase ascendante,
   - en ce qu'ils sont inducteurs, chez le singe Saimiri Sciureus splenectomisés, de la production d'anticorps protecteurs contre les parasites du paludisme dans les conditions suivantes :
   - les singes sus-mentionnés sont inoculés trois fois à trois semaines d'intervalle avec les polypeptides par injections sous-cutanées
   - trois semaines après la troisième injection, les animaux sont infectés avec 50.10⁶ globules rouges parasités par la souche de P. falciparum susmentionnée, par voie intraveineuse,
   - détection, 15 jours plus tard, d'une proportion de globules rouges parasités chez le singe susmentionné, inférieure à 10 %.

2. Composition selon la revendication 1, caractérisée par des polypeptides présentant des poids moléculaires moyens de l'ordre de 75.000 ± 5.000 daltons.

3. Composition selon la revendication 1 ou la revendication 2, caractérisée par le fait qu'elle est constituée en majeure partie par un polypeptide ayant l'un des poids moléculaires suivants : 72.000, 75.000, 76.000, 80.000, 83.000, 85.000 ou 90.000 daltons.

4. Composition selon la revendication 1, caractérisée par des polypeptides présentant des poids moléculaires moyen de l'ordre de 100.000 ± 10.000 daltons.

5. Composition selon la revendication 1 ou la revendication 4, caractérisée par le fait qu'elle est constituée en majeure partie par un polypeptide ayant l'un des poids moléculaires suivants : 90.000, 95.000, 96.000, 100.000, 105.000, 110.000, 115.000, 120.000 et 130.000 daltons.

6. Composition selon l'une quelconque des revendications 1 à 5, caractérisée par le fait qu'elle est constituée de polypeptides ayant des poids moléculaires compris entre 70.000 et 85.000, capables d'incorporer métaboliquement de la méthionine $^{35}$S.

7. Composition selon l'une quelconque des revendications 1 à 5, caractérisée par le fait qu'elle est constituée de polypeptides ayant des poids moléculaires compris entre 90.000 et 120.000, capables d'incorporer métaboliquement de la méthionine $^{35}$S.

8. Composition selon la revendication 1, caractérisée en ce qu'elle contient des polypeptides de poids moléculaires 90.000 réagissant avec des anticorps monoclonaux secrétés par l'hybridome déposé à la C.N.C.M. sous le n° I-271.

9. Composition selon l'une quelconque des revendications 1 à 8, caractérisée en ce qu'elle contient des polypeptides reconnus par les anticorps protecteurs contenus dans un sérum protecteur prélevé chez le singe Saimiri Sciureus immunisé, du fait d'une infection antérieure par la souche de P. falciparum FUPC I-212, le prélèvement étant intervenu dans un délai de 30 à 90 jours à compter de la fin de l'infection aiguë.

10. Composition selon l'une quelconque des revendications 1 à 9, caractérisée en ce que les polypeptides immunogènes qu'elle contient sont constitués par des polypeptides "intacts" en ce sens qu'ils ne consistent pas en des fragments de protéines membranaires ayant initialement eu des poids moléculaires plus élevés, synthétisés au stade des schizontes et ayant subi une digestion intracellulaire.

11. Composition selon la revendication 2, caractérisée par un polypeptide immunogène de poids moléculaire moyen 50.000 qui n'incorpore pas biologiquement la $^{35}$S-méthionine.

12. Procédé pour préparer une composition immunogène contenant un ou plusieurs polypeptides contenus dans des parasites du paludisme, ces polypeptides étant plus particulièrement aptes à réagir avec des anticorps protecteurs provenant de singes résistant au parasites humains du paludisme et notamment à des espèces de Plasmodium falciparum , caractérisé en ce que, partant d'une solution contenant à l'état dissous la majeure partie des structures cellulaires et des constituants protéiniques d'un parasite malarique, notamment appartenant à l'espèce Plasmodium , on sépare et on récupère à partir de cette solution ceux des polypeptides qui présentent notamment des poids moléculaires moyens s'étageant d'environ 50.000 à environ 140.000, qui sont capables à la fois d'induire la production d'anticorps protecteurs contre des parasite infectieux contre l'homme et/ou le singe et d'être reconnus par des anticorps protecteurs obtenus à partir de singes immunisés.

13. Procédé selon la revendication 12, caractérisé en ce que la solution de départ résulte du traitement du parasite malarique, notamment du type Plasmodium , tel que Plasmodium falciparum avec une solution d'un détergent, tel que le dodécylsulfate de sodium, susceptible d'induire la dissolution de la susdite majeure partie des structures cellulaires et des constituants protéiniques du parasite.

14. Procédé selon la revendication 12, ou la revendication 13, caractérisé en ce que l'on sépare plus particulièrement les polypeptides ayant des poids moléculaires moyens de 75.000 ± 5.000.

15. Procédé selon la revendication 13 ou la revendication 14, caractérisé en ce que l'on sépare plus particulièrement les polypeptides ayant des poids moléculaires moyen de 100.000 ± 10.000.

16. Procédé selon la revendication 12 ou la 13, caractérisé en ce que l'on sépare plus particulièrement les polypeptides ayant des poids moléculaires de l'ordre de 50.000.

17. Procédé selon l'une quelconque des revendications 12 à 16, caractérisé en ce qu'il comprend au moins une gel-filtration, électrophorèse ou analogue, et la récupération des polypeptides présentant les poids moléculaires correspondants.

18. Procédé selon la revendication 12 ou la revendication 13, caractérisé par la mise en contact de la solution de départ avec des anticorps protecteurs fixés sur un support insoluble et l'élution du complexe formé entre les anticorps fixés et les antigènes correspondants.

19. Procédé selon la revendication 17 et la revendication 18, caractérisé en ce qu'il comprend à la fois une gel-filtration, l'électrophorèse ou analogue, et une mise en contact avec des anticorps, la gelfiltration ou l'électrophorèse précédant la mise en contact des fractions récupérées avec les anticorps, ou vice-versa.

20. Procédé selon la revendication 18 ou la revendication 19, caractérisé en ce que les anticorps protecteurs fixés sont remplacés par des anticorps monoclonaux préalablement formés contre l'un des polypeptides réagissant avec les susdits anticorps protecteurs.

21. Procédé de diagnostic in vitro de la présence d'anticorps anti-paludéens dans un sérum comprenant les étapes suivantes :
    - dépôt de quantités déterminées d'un polypeptide de la composition selon l'une quelconque des revendications 1 à 11 dans les puits d'une microplaque du type de celle utilisée pour la mise en oeuvre de la méthode ELISA ;
    - introduction de dilutions croissantes du sérum contenant, le cas échéant, les anticorps à détecter, voire à doser dans les puits de cette microplaque ;
    - incubation de la microplaque et de son contenu ;
    - lavage poussé de la microplaque avec un tampon approprié ;
    - introduction d'anticorps marqués dirigés contre les premiers, le marquage étant fait au moyen d'une molécule fluorescente ou d'une enzyme capable d'hydrolyser un substrat et choisie parmi celles auxquelles correspondent des substrats dont l'hydrolyse se manifeste par une radiation de longueur d'onde donnée,
    - mesure, selon le cas, de l'intensité de fluorescence ou de la variation d'absorbance et
    - détermination , de préférence vis-à-vis de mesures semblables faite vis-à-vis d'une témoin de la teneur en anticorps du sérum étudié.

22. Nécessaire ou "kit" de diagnostic in vitro de la présence d'antigènes paludéens dans un sérum comprenant :
    - l'un des polypeptides protecteurs contenus dans la composition selon l'une quelconque des revendications 1 à 11, ce polypeptide étant marqué, par une enzyme ou par un composé fluorescent ;
    - des anticorps spécifiques du polypeptide utilisé ;
    - des tampons et substrats éventuellement nécessaires à la révélation du marqueur.

23. Préparation vaccinante contre la malaria formée par l'association de la composition immunogène selon l'une quelconque des revendications 1 à 11 et d'un véhicule pharmaceutique approprié au mode d'administration choisi, notamment par voie parentérale.

24. Composition d'anticorps purifiés, caractérisés par leur capacité de réaction avec les polypeptides immunogènes contenus dans une composition conforme à l'une quelconque des revendications 1 à 11.

**Claims**

1. Immunogenic composition, containing one or several polypeptides chosen among polypeptides having molecular weights of the order of 50.000 to 140.000, these polypeptides being characterized :
    - in that they are recognized by protective antibodies against malaria parasites, these antibodies being contained in sera, ascites or other immunoglobulin compositions, originating from Saimiri Sciureus monkeys made resistant to the P.falciparum strain deposited with the CNCM under the number I-212, this resistance being obtained by infection of these monkeys with said P.falciparum strain (made virulent by successive passages of said strain in several splenectomized Saimiri Sciureus monkeys) followed by a treatment with quinine, these sera, ascites or other immunoglobulin compositions, being capable by in vivo passive transfer to sensitive splenec-

14

tomized recipient monkeys, to protect them against infection by said P.falciparum strain, when these have previously received the injection of $50.10^6$ of cells parasitized by said P.falciparum strain, these cells being themselves obtained from splenectomized and infected by said P.falciparum strain Saimiri Sciureus monkeys, and at the moment when the infection was acute and in ascending phase,

- in that they are inducers, in splenectomized Saimiri Sciureus monkey, of the production of protective antibodies against malaria parasites in the following conditions :
- the above mentioned monkeys are inoculated three times at an interval of three weeks with the polypeptides by subcutaneous injections,
- three weeks after the third injection, the animals were infected with $50.10^6$ red blood cells parasitized by the above mentioned P.falciparum strain by intravenous route,
- detection, 15 days later, of a proportion of parasitized red blood cells in the above mentioned monkey, lower than 10 %.

2. Composition according to claim 1 characterized by polypeptides presenting average molecular weights of about $75.000 \pm 5.000$.

3. Composition according to claim 1 or claim 2, characterized by the fact that it is constituted in major part by a polypeptide having one of the following molecular weights : 72.000, 75.000, 76.000, 80.000, 83.000, 85.000 or 90.000 daltons.

4. Composition according to claim 1 characterized by polypeptides presenting average molecular weights of about $100.000 \pm 10.000$.

5. Composition according to claim 1 or claim 4 characterized by the fact that it is constituted in major part by a polypeptide having one of the following molecular weights : 90.000, 95.000, 96.000, 100.000, 105.000, 110.000, 115.000, 120.000 and 130.000 daltons.

6. Composition according to anyone of claims 1 to 5, characterized by the fact that it is constituted of polypeptides having molecular weights comprised between 70.000 and 85.000, capable of metabolically incorporating $^{35}$S methionine.

7. Composition according to anyone of claims 1 to 5, characterized by the fact that it is constituted of polypeptides having molecular weights comprised between 90.000 and 120.000, capable of metabolically incorporating $^{35}$S methionine.

8. Composition according to claim 1, characterized in that it contains polypeptides of molecular weights 90.000 reacting with monoclonal antibodies secreted by the hybridoma deposited at the CNCM under n° I-271.

9. Composition according to anyone of claims 1 to 9, characterized by the fact that it contains polypeptides recognized by protective antibodies contained in a protective serum collected from immunized Saimiri sciureus monkey, as a result of previous infection by FUPC I-212 P.falciparum strain, said sample being collected within from 30 to 90 days from the end of the acute infection.

10. Composition according to anyone of claims 1 to 9, characterized by the fact that the immunogenic polypeptides which it contains are constituted by "intact" polypeptides, in the sense that they do not consist of fragments of membrane proteins which previously had higher molecular weights, synthesized at the stage of schizonts and which underwent intracellular digestion.

11. Composition according to claim 2, characterized by an immunogenic polypeptide of average molecular weight of 50.000 which do not incorporate biologically $^{35}$S methionine.

12. Process for preparing an immunogenic composition containing one or several polypeptides contained in malaria parasites, these polypeptides being more particularly able to react with protective antibodies originating from monkeys resistant to human parasites of malaria and particularly to species of Plasmodium falciparum , characterized in that starting from a solution containing at the soluble state the major part of the cellular structures and of the parasite proteinic constituents from malaria parasite, particularly of the Plasmodium type, one separates and recovers from this solution those of the polypeptides which present notably average molecular weights ranging from about 50.000 to about 140.000, which are capable of inducing the production of antibodies protective against parasites which are infectious to man and/or to monkey as well and of being recognized by protective antibodies obtained from immunized monkeys.

13. Process according to claim l2, characterized in that the starting solution results from the treatment of the malaria parasite, notably of the plasmodium type, such as Plasmodium falciparum with a solution of a detergent, such as sodium dodecylsulfate, liable to induce the dissolution of the aforesaid major part of the cellular structures and of the parasite proteinic constituents.

14. Process according to claim 12, or claim 13, characterized in that one separates more particularly the polypeptides having average molecular weights of 75.000 ± 5.000.

15. Process according to claim 13, or claim 14, characterized in that one separates more particularly the polypeptides having average molecular weights of 100.000 ± 10.000.

16. Process according to claim 12, or claim 13, characterized in that one separates more particularly the polypeptides having average molecular weights of about 50.000.

17. Process according to anyone of claims 12 to 16, characterized in that it comprises at least a gel filtration, electrophoresis or alike, and the recovery of polypeptides having the corresponding molecular weights.

18. Process according to claim 12, or claim 13, characterized by the placing into contact of the starting solution with protective antibodies fixed on a non-soluble support and the elution of the complex formed between the fixed antibodies and the corresponding antigens.

19. Process according to claim 17 and claim 18, characterized in that it comprises both a gel filtration, electrophoresis or alike, and a placing into contact with antibodies, the gel filtration or electrophoresis preceding the placing into contact of the recovered fractions with the antibodies, or conversely.

20. Process according to claim 18, or claim 19, characterized in that the fixed protective antibodies are replaced by monoclonal antibodies previously formed against one of the polypeptides reacting with the above mentioned protective antibodies.

21. Process for the in vitro diagnosis of the presence of antimalarial antibodies in a serum comprising the following steps :

- deposit of determined amounts of a polypeptide of the composition according to anyone of claims 1 to 11, in the wells of a microplate of the type used in ELISA method ;
- introduction of increasing dilutions of the serum possibly containing the antibodies to be detected or to be titrated in the wells of this microplate ;
- incubating the microplate and its content ;
- thorough washing of the microplate with an appropriate buffer ;
- introduction of labelled antibodies directed against the first ones, the labelling having been made by means of a fluorescent molecule or of an enzyme able to hydrolysate a substrate and chosen among those to which correspond substrates, the hydrolysis of which is revealed by a given wave length radiation ;
- measuring of the fluorescence intensity or of the absorbance variation, as appropriate, and
- determination, preferably with respect to similar measures carried out with respect to a control, of the content in antibodies of the studied serum.

22. Kit for in vitro diagnosis of the presence of malarial antigens in a serum comprising :
- one of the protective polypeptides contained in the composition according to anyone of claims 1 to 11, this polypeptide being labelled, by an enzyme or a fluorescent compound ;
- antibodies specific of the used polypeptides ;
- buffers and substrates, if appropriate useful for revealing the label.

23. Vaccinating preparation against malaria formed by the association of the immunogenic composition according to anyone of claims 1 to 11 and a pharmaceutical vehicle appropriate to the administration route chosen, notably to parenteral route.

24. Composition of purified antibodies, characterized by their capacity of reaction with immunogenic polypeptides contained in a composition conform to anyone of claims 1 to 11.

**Ansprüche**

1. Immunogene Zusammensetzung, enthaltend ein oder mehrere Polypeptide, die unter Polypeptiden mit Molekulargewichten der Größenordnung von 50.000 bis 140.000 ausgewählt sind, wobei diese Polypeptide dadurch gekennzeichnet sind:

- daß sie durch Schutzantikörper gegen die Parasiten der Malaria erkannt werden, wobei diese Antikörper in Seren, Ascites-Flüssigkeiten oder anderen Zusammensetzungen von Immunglobulinen enthalten sind, die von Affen Saimiri Sciureus stammen, die gegenüber dem Stamm P. falciparum resistent gemacht wurden, der bei dem C.N.C.M. unter der Nummer I-212 hinterlegt ist, wobei diese Resistenz durch Infektion dieser Affen durch den genannten Stamm von P. falciparum (der durch aufeinanderfolgende Durchgänge des Stammes bei mehreren Affen Saimiri Sciureus, denen die Milz entfernt wurde, virulent geworden ist), gefolgt von einer Behandlung mit Chinin, wobei diese Seren, Ascites-Flüssigkeiten oder andere Zusammensetzungen von Immunglobulinen durch passive Übertragung in vivo auf empfindliche Empfänger-Affen ohne Milz fähig sind, sie gegen Infektion durch den Stamm von P. falciparum zu schützen, wenn diese vorher die Injektion von 50.10$^6$ mit dem genannten Stamm von P. falciparum parasitierten Zellen erhalten haben, wobei die Zellen ihrerseits ausgehend von Affen Saimiri Sciureus erhalten wurden, denen die Milz entfernt wurde und die durch den genannten Stamm von P. falciparum infiziert wurden, und zu dem Zeitpunkt, wo die Infektion akut und in aufsteigender Phase war,

- daß sie bei Affen Saimiri Sciureus, denen die Milz entfernt wurde, Induktoren der Erzeugung von Schutzantikörpern gegen die Parasiten der Malaria in den folgenden Bedingungen sind: die oben erwähnten Affen werden drei Mal in dreiwöchigem Intervall mit den Polypeptiden durch subkutane Injektionen geimpft, drei Wochen nach der dritten Injektion werden die Tiere mit 50.10$^6$, durch den oben erwähnten Stamm von P. falciparum parasitierten, roten Blutkörperchen intravenös infiziert, 15 Tage später wird bei den oben erwähnten Affen ein Anteil von verseuchten roten Blutkörperchen nachgewiesen, der kleiner als 10 % ist.

2. Zusammensetzung nach Anspruch 1, gekennzeichnet durch Polypeptide mit mittleren Molekulargewichten der Größenordnung von 75.000 ± 5.000 Dalton.

3. Zusammensetzung nach Anspruch 1 oder Anspruch 2, dadurch gekennzeichnet, daß sie zum größeren Teil durch ein Polypeptid mit einem der folgenden Molekulargewichte gebildet wird: 72.000, 75.000, 76.000, 80.000, 83.000, 85.000 und 90.000 Dalton.

4. Zusammensetzung nach Anspruch 1, gekennzeichnet durch Polypeptide mit mittleren Molekulargewichten der Größenordnung von 100.000 ± 10.000 Dalton.

5. Zusammensetzung nach Anspruch 1 oder Anspruch 4, dadurch gekennzeichnet, daß sie zum größeren Teil durch ein Polypeptid mit einem der folgenden Molekulargewichte gebildet wird: 90.000, 95.000, 96.000, 100.000, 105.000, 110.000, 115.000, 120.000 und 130.000 Dalton.

6. Zusammensetzung nach einem der Ansprüche 1 bis 5, dadurch gekennzeichnet, daß sie von Polypeptiden mit Molekulargewichten gebildet ist, die zwischen 70.000 und 85.000 liegen, und in der Lage sind, metabolisch das Methionin $^{35}$S aufzunehmen.

7. Zusammensetzung nach einem der Ansprüche 1 bis 5, dadurch gekennzeichnet, daß sie von Polypeptiden mit Molekulargewichten zwischen 90.000 und 120.000 gebildet wird, die in der Lage sind, metabolisch Methionin $^{35}$S aufzunehmen.

8. Zusammensetzung nach Anspruch 1, dadurch gekennzeichnet, daß sie Polypeptide mit Molekulargewichten von 90.000 enthält, die mit monoklonalen Antikörpern reagieren, die von dem Hybridom ausgeschieden werden, das bei dem C.N.C.M. unter der Nr. I-271 hinterlegt ist.

9. Zusammensetzung nach einem der Ansprüche 1 bis 8, dadurch gekennzeichnet, daß sie Polypeptide enthält, die von den Schutzantikörpern erkannt werden, die in einem Schutzserum enthalten sind, das bei immunisierten Affen Saimiri Sciureus entnommen ist, aufgrund einer früheren Infektion durch den Stamm von P. falciparum FUPC I-212, wobei die Entnahme in einem Zeitraum von 30 bis 90 Tagen, gerechnet vom Ende der akuten Infektion, durchgeführt wurde.

10. Zusammensetzung nach einem der Ansprüche 1 bis 9, dadurch gekennzeichnet, daß die immunogenen Polypeptide, die sie enthält, von in diesem Sinne "intakten" Polypeptiden gebildet werden, daß sie nicht aus Fragmenten von Membranproteinen bestehen, die vorher höhere Molekulargewichte aufwiesen, synthetisiert im Stadium der Schizonten und nachdem sie einen intrazellulären Abbau durchgemacht haben.

11. Zusammensetzung nach Anspruch 2, gekennzeichnet durch ein immunogenes Polypeptid mit einem mittleren Molekulargewicht von 50.000, das biologisch das $^{35}$S-Methionin nicht aufnimmt.

12. Verfahren zur Herstellung einer immunogenen Zusammensetzung mit einem oder mehreren in den Erregern der Malaria enthaltenen Polypeptiden, wobei die Polypeptide insbesondere geeignet sind, mit Schutzantikörpern zu reagieren, die von gegenüber den menschlichen Erregern der Malaria und insbesondere den Arten von Plasmodium falciparum resistenten Affen stammen, dadurch gekennzeichnet, daß ausgehend von einer Lösung, die im gelösten Zustand den größeren Teil der Zellstrukturen und der proteinischen Bestandteile eines Malariaerregers, insbesondere der Art Plasmodium angehörend, man ausgehend von dieser Lösung diejenigen der Polypeptide trennt und wieder-gewinnt, die insbesondere mittlere Molekulargewichte darstellen, die sich zwischen etwa 50.000 und etwa 140.000 erstrecken, die in der Lage sind, gleichzeitig die Produktion von Schutzantikörpern gegen die Infektionserreger beim Menschen und/oder beim Affen zu induzieren und durch die, ausgehend von immunisierten Affen erhaltenen, Schutzantikörper erkannt zu werden.

13. Verfahren nach Anspruch 12, dadurch gekennzeichnet, daß die Ausgangslösung von der Behandlung des Malariaerregers, insbesondere des Typs Plasmodium , beispielsweise des Plasmodium falciparum , mit einer Lösung eines Detergens stammt, beispielsweise Natriumdodecylsulfat, das in der Lage ist, die Auflösung des genannten größeren Teils der Zellstrukturen und der proteinischen Bestandteile des Erregers zu induzieren.

14. Verfahren nach Anspruch 12 oder Anspruch 13, dadurch gekennzeichnet, daß man insbesondere die Polypeptide mit mittleren Molekulargewichten von 75.000 ± 5.000 trennt.

15. Verfahren nach Anspruch 13 oder Anspruch 14, dadurch gekennzeichnet, daß man insbesondere die Polypeptide mit mittleren Molekulargewichten 100.000 ± 10.000 trennt.

16. Verfahren nach Anspruch 12 oder Anspruch 13, dadurch gekennzeichnet, daß man insbesondere die Polypeptide mit Molekulargewichten der Größenordnung von 50.000 trennt.

17. Verfahren nach einem der Ansprüche 12 bis 16, dadurch gekennzeichnet, daß es mindestens eine Gel-Filtration, Elektrophorese o.dgl. umfaßt sowie die Wiedergewinnung der die entsprechenden Molekulargewichte darstellenden Polypeptide.

18. Verfahren nach Anspruch 12 oder Anspruch 13, dadurch gekennzeichnet, daß die Ausgangslösung mit auf einen unlösbaren Träger fixierten Schutzantikörpern in Kontakt gebracht wird, und daß der zwischen den fixierten Antikörpern und den entsprechenden Antigenen gebildete Komplex ausgewaschen wird.

19. Verfahren nach Anspruch 17 und Anspruch 18, dadurch gekennzeichnet, daß es gleichzeitig eine Gel-Filtration, Elektrophorese o.dgl. und eine Kontaktierung mit den Antikörpern umfaßt, wobei die Gel-Filtration oder Elektrophorese der Kontaktierung der wiedergewonnenen Fraktionen mit den Antikörpern vorausgeht, oder umgekehrt.

20. Verfahren nach Anspruch 18 oder Anspruch 19, dadurch gekennzeichnet, daß die fixierten Schutzantikörper durch monoklonale Antikörper ersetzt werden, die vorher gegen eines der mit den genannten Schutzantikörpern reagierenden Polypeptide gebildet wurden.

21. Verfahren der in vitro -Diagnose des Vorhandenseins von Antimalaria-Antikörpern in einem Serum umfassend die folgenden Verfahrensschritte:
- bestimmte Mengen eines Polypeptids der Zusammensetzung nach einem der Ansprüche 1 bis 11 wird in die Vertiefungen einer Mikrotiterplatte eingebracht, derart, wie sie für die Durchführung des ELISA-Tests verwendet wird;
- wachsende Verdünnungen des Serums, das ggf. die nachzuweisenden Antikörper enthält, werden in die Vertiefungen die Mikrotiterplatte dosiert eingebracht;
- die Mikrotiterplatte und ihr Inhalt wird inkubiert;
- die Mikrotiterplatte wird mit einem geeigneten Puffer gewaschen;
- es werden gegen die ersten gerichtete markierte Antikörper eingeführt, wobei die Markierung mit Hilfe eines fluoreszierenden Moleküls oder eines Enzyms geschieht, das fähig ist, ein Substrat zu hydrolysieren, ausgewählt unter denen, welchen die Substrate entsprechen, deren Hydrolyse sich durch eine Strahlung einer gegebenen Wellenlänge zeigt,
- je nach Fall wird die Intensität der Fluoreszenz oder die Veränderung der Absorption gemessen und
- es wird der Gehalt an Antikörpern des untersuchten Serums bestimmt, vorzugsweise gegenüber ähnlichen Messungen, die mit einer Referenzprobe durchgeführt wurden.

22. Kit für die in vitro -Diagnose des Vorhandenseins von Malaria-Antigenen in einem Serum, enthaltend:
- eines der in der Zusammensetzung nach einem der Ansprüche 1 bis 11 enthaltenen Schutzpolypeptide, wobei das Polypeptid durch ein Enzym oder durch eine fluoreszierende Komponente markiert ist;
- für das verwendete Polypeptid spezifische Antikörper;
- Puffer und Substrate, die ggf. zur Entwicklung des Markers notwendig sind.

23. Impfzusammensetzung gegen die Malaria, gebildet durch die Zuordnung der immunogenen Zusammensetzung nach einem der Ansprüche 1 bis 11 und eines pharmazeutischen Transportmittels, das für die gewählte Verabreichungsart geeignet ist, insbesondere auf parenteralem Wege.

24. Zusammensetzung von gereinigten Antikörpern, gekennzeichnet durch ihre Reaktionsfähigkeit mit den in einer Zusammensetzung nach einem der Ansprüche 1 bis 11 enthaltenen immunogenen Polypeptiden.